# EUROPEAN PATENT SPECIFICATION

(11) **EP 1 795 142 B1**
(45) Date of publication and mention of the grant of the patent: **11.06.2008**
(21) Application number: 05025626.2
(22) Date of filing: 24.11.2005
(51) Int. Cl.: A61B 19/00

(54) **Medical tracking system using a gamma camera**
Medizinisches Referenzierungssystem mit gamma-Kamera
Système de suivi médical utilisant une camera pour les rayons gamma

(43) Date of publication of application: 13.06.2007
(73) Proprietor: BrainLAB AG, 85551 Kirchheim/Heimstetten (DE)
(72) Inventor: Keglovich, Mike, St. Petersburg, Florida 33710 (US); Pedain, Christoph, Dr., 81667 Munich (DE)
(74) Representative: Schwabe - Sandmair - Marx

(56) References cited:
- EP-A- 1 554 987
- WO-A-01/79884
- WO-A-20/05012945
- US-A1- 2004 015 075
- US-B1- 6 587 710

## Description

The present invention relates to the tracking of instruments and objects in a medical environment. A medical navigation and tracking system is, for example, disclosed in DE 196 39 615 C2. Various forms of intraoperative imaging coupled with navigation are becoming standardized in today's medical environment. This trend will continue to grow in various fields, including tumor/tissue resection, patient positioning, and confirmation of planned trajectories. Some current forms of intraoperative imaging include CT and MRI; however, these diagnostic tools and subsequent images are neither real time nor precise enough to locate certain abnormalities, especially in deep tissue areas. Although ultrasound is real time intraoperative imaging, depth and resolution of images can be limited with the current technology.

US 2004/0015075A1 discloses a system for calculating a position of a radioactivity emitting source in a system-of-coordinates, the system comprising a radioactive emission detector; a position tracking system being connected to and/or communicating with the radioactive emission detector; and a data processor being designed and configured for receiving data inputs from the position tracking system and from the radioactive emission detector and for calculating the position of the radioactivity emitting source in the system-of-coordinates. Further disclosed is a surgical instrument being connected to and/or communicating with a second position tracking system for tracking a position of said instrument in a second position tracking system and at least one data processor being designed and configured for receiving data inputs from said first position tracking system, said radioactive emission detector and said second position tracking system and for calculating the position of the surgical instrument and the pharmaceutical uptaking portion of the body component in a common system-of - coordinates.

It is an object of the present invention to provide medical tracking or navigation with a functional real time intraoperative imaging.

This object is achieved by a medical tracking system according to claim 1 . The subclaims define advantageous embodiments of the invention.

In accordance with the invention a medical tracking system comprises a camera based localisation system having a video camera system or an infrared camera system or both spatially localising medical instruments and a computer assigning absolute and/or relative positions of said instruments in a coordinate system said camera system defining said coordinate system. Said tracking system further comprises at least one localisable gamma camera sensitive for detecting gamma radiation emitted from a tracer material.

The medical tracking system further comprises instrument markers detectable by said gamma camera, said gamma camera carrying tracking markers by means of which it can be spatially localised and/or identified in said tracking system.

Furthermore, an instrument or a part of an instrument is localised by detecting a tracer material irradiating gamma energy, in particular a radioactive material on said instrument by means of said gamma camera.

In other words, an imaging modality using gamma rays combined with a tracking/navigation system can be used intraoperatively. Gamma ray technology can provide benefits not previously offered with other intraoperative treatment pathways. Gamma rays are one of three sources of radioactive radiation. The other two sources are alpha and beta rays, each having distinctly different properties and characteristics than gamma rays. Some characteristics of gamma radiation include the following:
- it consists of pure energy;
- it is photon based;
- it is able to travel long distances and penetrate through objects easily.

On this basis, a gamma camera can provide functional real time intraoperative imaging. Advantageously, the invention thus allows a surgeon to see anatomical structures, soft tissue and abnormalities as they appear within the body at that moment in time. Additionally, the imaging can display the surgical instrument within the region of interest and the surgeon can also verify complete resection of tissue/tumors

Costly operating room time is not spent repositioning patients into an intraoperative CT scanner or MRI during intraoperative imaging. When using gamma camera/ray technology, the device is portable, similar to an adaptable surgical tool and can obtain images much faster in real time.

According to a further embodiment, the medical tracking system comprises an electromagnetic localisation system creating an electromagnetic field in which objects can be localised, said electromagnetic field defining said or a coordinate system.

Said or a coordinate system may be defined on the basis of determined positions of said gamma camera and/or one or more of said localised instruments.

The invention also relates to a medical navigation system comprising a tracking system as has been described above. Such a medical navigation system may comprise a memory device storing images obtained by said gamma camera.

In one embodiment, such a medical navigation system comprises an image output for displaying images obtained by said gamma camera, in particular together with one or more of the following:
- spatially assigned or registered patient image data, such as CT or MR data;
- spatially assigned or registered instruments or their positional data;
- instruments localised by said gamma camera; and
- said gamma camera itself.

Further disclosed is a tracking method in a medical environment, said method comprising the spatial localisation of medical instruments and the computer assisted assignment of absolute and/or relative positions of said instruments in a coordinate system, characterised by the localisation of a at least one localisable gamma camera sensitive for detecting gamma radiation emitted from a tracer material.

At least one of the following coordinate systems can be used:
- a coordinate system defined by a camera based localisation system having a video camera system or an infrared camera system or both;
- a coordinate system defined by an electromagnetic localisation system creating an electromagnetic field in which objects can be localised; and
- a coordinate system defined on the basis of determined positions of said gamma camera and/or one or more of said localised instruments.

Also, at least two of said coordinate systems may be used in combination and correlated with each other in the localisation of instruments and/or said gamma camera in order to create a multi-modal tracking system.

In accordance with one embodiment, an image or images made by the gamma camera are used in an image guided surgery system or a medical navigation system or a surgical/medical planning system performing or planning at least one of the following tasks:
- displaying images made by the gamma camera;
- storing images made by the gamma camera;
- creating patient image data sets from images made by the gamma camera;
- spatially assigning or registering previously acquired patient image data, such as CT or MR data to the images made by the gamma camera;
- spatially assigning or registering instruments or their positional data to the images made by the gamma camera;
- displaying said instruments localised by said gamma camera or said gamma camera itself in positional relationship to said image data sets created or acquired;
- displaying said tracer material in selectively enriched regions of interest, in particular together with information relevant to a medical procedure and/or with information relevant to the positioning of an instrument.

Any images made by the gamma camera may be used not only for real time navigation, but, of course, also for planning a subsequent therapy.

The following describes the invention in a still general, but more detailed, yet not limiting way:

Before the invention is carried out and independently from all steps of a disclosed method, radioactive tracer material is injected into the patient. This tracer material is selectively enriched based on properties of the different body portions, such as based on molecular binding.

The handheld gamma camera is positioned over the region of interest (ROI) of the patient. The radioactive tracer material emits energy within the ROI, and the camera collects the photons being from the tracer material and produces an image emitted from there. This image can now be displayed in a navigation system. The navigation system, in real time, can then reconstruct multiple images taken from different angles to a 3D image and can show the 3D image predicated on the spatial relation between the camera and patient location. Although not limited to this, the technology can be used in such modalities and treatments as breast tumors, melanoma, prostate cancer, kidney metastasis, and lung metastasis for precise diagnosis and tracking of the target volume during a surgical procedure.

The camera can as well be used to track instruments or markers introduced into the body even without. In that application, the gamma camera acts as an instrument tracking extension which gives the current system an x-ray vision. In this application, the position and orientation of the gamma camera is determined by the navigation system and the spatial information about the tracking markers is used to correlate the position of various markers with each other. This application is possible by using one or more gamma cameras, either mounted in a fixed and known location and orientation, or tracked with another tracking system.

The gamma camera can thus be used as a tracking system to track the instruments and/or the targeted area. This expands the application of the technology to a true simultaneous tracking technique for moving instruments and moving target areas.

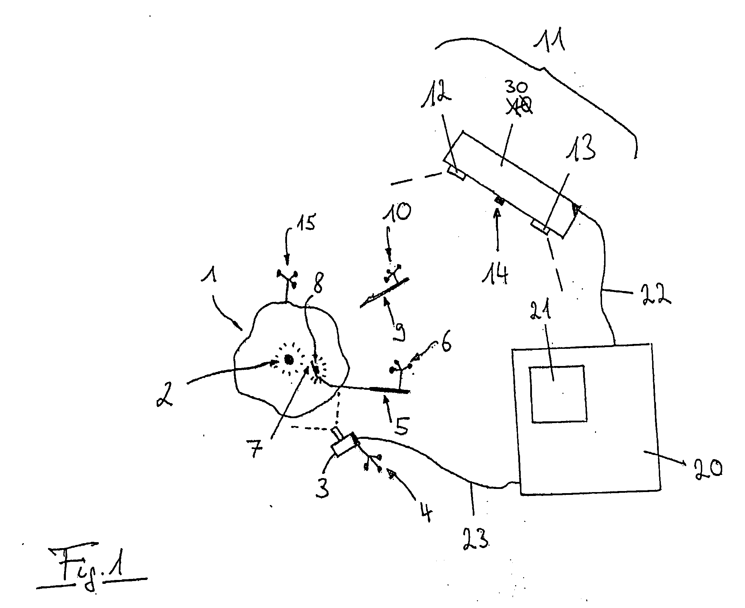

The invention is now further described by means of showing one particular embodiment and with reference to the attached single Figure 1. The invention may comprise each of the features disclosed herein independently or in any combination. Figure 1 shows a schematic representation of a tracking system in accordance with the present invention.

In Figure 1, reference numeral 1 designates a schematic representation of a patient's body part; 2 is a gamma radiant tracer material in a region of interest; 3 is a gamma camera; 4, 6 and 10 are arrays of reflective spheres; 5 is an instrument with a flexible tip section 7 and a tracer material 8 on said tip section; 9 is a rigid instrument such as a scalpel; 30 is a camera holder; 11 designates an infrared tracking system; 12 and 13 are stereoscopic infrared tracking cameras; 14 is a source of infrared light; 15 is a reflective patient marker array; 20 is a navigation system with a display 21 to which infrared tracking system 11 and gamma camera 3 are connected via lines 22 and 23, respectively. Gamma camera 3, instrument 5 and scalpel 9 are tracked by infrared tracking system 11 and navigated by navigation system 20 as is, for example, disclosed in DE 196 39 615 C2.

Injection of radioactive tracer material 2 into the patient 1 takes place approximately 45 minutes to 24 hours prior to surgery. The photons within the region of interest are reflected back to the camera 3 by use of this radioactive tracer material 2. The amount is not large enough to negatively affect the medical team participating in the procedure, although protection through the use of lead vests and protective glasses is available.

Reflective spheres 15 and 4 are positioned in the patient field and on the pre-calibrated gamma camera 3 via universal adapters. Diagnostic images are loaded into the navigation system 20. The patient is registered using known methods, and the spatial relationships between instruments 5 and 9, gamma camera 3, and patient 1 are coordinated via the navigation software through the infrared reflectors 4, 6, 10, 15 and displayed on the navigation display, i.e. monitor 21.

Since pre-calibrated gamma camera 3 is tracked, i.e. localised by tracking system 11 through reflective array 4, the content of the images obtained by said camera can also be positionally assigned and those images can supplement the diagnostic image information provided by previous imaging and loaded into the navigation system 20. The gamma camera images are obtained in real time, and surgeons can view anatomical regions and shapes and are able to identify abnormalities as they appear within the body intraoperatively.

Additionally, the surgeon can view surgical instruments 5 and 9 approaching the tissue he plans to extract and verify complete resection. In the case of instrument 5 which has a flexible tip section 7, the infrared tracking system may not be able to provide exact information about the tip's location in the body portion 2. To make up for this lack of information, instrument 5 is equipped with some tracer material 8 at its tip section 7. Thus, said tip section 7 can be localised by gamma camera 3 and represented positionally correct by the navigation system.

## Claims

1. Medical tracking system comprising a camera based localisation system (11, 20) having a video camera system (11) or an infrared camera system (11) or both, said camera system (11) defining a coordinate system , at least one localisable gamma camera (3) sensitive for detecting gamma radiation emitted from a tracer material (2, 8) and at least one additional instrument (5, 9), the at least one localizable gamma camera (3) and the at least one additional medical instrument (5, 9) being localizable in the camera based system (11, 20) a computer (20) assigning absolute and/or relative positions of said instruments in said coordinate system, **characterized in that** the medical instrument (5, 9) additionally comprises instrument markers (8) detectable by said gamma camera (3).

2. The medical tracking system of claim 1, comprising an electromagnetic localisation system creating an electromagnetic field in which objects can be localised, said electromagnetic field defining said or a coordinate system.

3. The medical tracking system of one of the claims 1 or 2, wherein said or a coordinate system is defined on the basis of determined positions of said gamma camera (3) and/or one or more of said localised instruments (5, 9).

4. The medical tracking system of one of the claims 1 to 3, wherein said gamma camera (3) carries tracking markers by means of which it can be spatially localised and/or identified in said tracking system.

5. A medical navigation system comprising a tracking system of one or more of the preceding claims.

6. The medical navigation system of claim 5, comprising a memory device storing images obtained by said gamma camera (3).

7. The medical navigation system of claim 5 or claim 6, comprising an image output for displaying images obtained by said gamma camera (3), in particular together with one or more of the following:
- spatially assigned or registered patient image data, such as CT or MR data;
- spatially assigned or registered instruments or their positional data;
- instruments localised by said gamma camera; and
- said gamma camera itself.

## Patentansprüche

1. Medizinisches Referenzierungssystem, mit einem kameragestützten Ortungssystem (11, 20) mit einem Videokamerasystem (11) und/oder einem Infrarotkamerasystem (11), wobei das Kamerasystem (11) ein Koordinatensystem definiert, mindestens einer ortbaren Gamma-Kamera (3), welche von einem Indikatormaterial (2, 8) emittierte Gamma-Strahlung erkennen kann und mindestens einem zusätzlichen Instrument (5, 9), wobei die mindestens eine ortbare Gamma-Kamera (3) und das mindestens eine zusätzliche medizinische Instrument (5, 9) im kameragestützten System (11, 20) ortbar sind mit einem Computer (20), der absolute und/oder relative Positionen der Instrumente im Koordinatensystem zuordnet, **dadurch gekennzeichnet, dass** das medizinische Instrument (5, 9) zusätzlich durch die Gamma-Kamera (3) detektierbare Instrumentenmarker (8) umfasst.

2. Medizinisches System gemäß Anspruch 1, mit einem elektromagnetischen Ortungssystem, das ein elektromagnetisches Feld erzeugt, in welchem Objekte geortet werden können, wobei das elektromagnetische Feld das oder ein Koordinatensystem definiert.

3. Medizinisches System gemäß einem der Ansprüche 1 oder 2, wobei das oder ein Koordinatensystem auf der Basis von bestimmten Positionen der Gamma-Kamera (3) und/oder eines oder mehrerer der georteten Instrumente (5, 9) definiert wird.

4. Medizinisches System gemäß einem der Ansprüche 1 bis 3, wobei die Gamma-Kamera (3) Tracking-Marker aufweist, durch welche sie im Referenzierungssystem räumlich geortet und/oder identifiziert werden kann.

5. Medizinisches Navigationssystem mit einem Referenzierungssystem gemäß einem oder mehrerer der vorhergehenden Ansprüche.

6. Medizinisches Navigationssystem gemäß Anspruch 5, mit einer Speichervorrichtung, welche durch die Gamma-Kamera (3) erhaltene Bilder speichert.

7. Medizinisches Navigationssystem gemäß Anspruch 5 oder 6, mit einer Bildausgabe zum Anzeigen von von der Gamma-Kamera (3) erhaltenen Bildern, insbesondere zusammen mit einem oder mehreren der folgenden Elemente:
- räumlich zugeordnete oder registrierte Patientenbilddaten, wie etwa CT- oder MR-Daten;
- räumlich zugeordnete oder registrierte Instrumente oder deren Positionsdaten;
- durch die Gamma-Kamera geortete Instrumente; und
- die Gamma-Kamera selbst.

## Revendications

1. Système de suivi médical comprenant un système de localisation (11, 20) à caméra comprenant un système de caméra vidéo (11) ou un système de caméra à infrarouge (11) ou les deux, ledit système de caméra (11) définissant un système de coordonnées, au moins une gamma-caméra localisable (3) sensible pour détecter le rayonnement gamma émis par un traceur (2, 8) et au moins un instrument supplémentaire (5, 9), la/les gamma-caméras localisables (3) et le/les instruments médicaux supplémentaires (5, 9) étant localisables dans le système (11, 20) à caméra, un ordinateur (20) attribuant des positions absolues et/ou relatives desdits instruments dans ledit système de coordonnées, **caractérisé en ce que** l'instrument médical (5, 9) comprend en outre des repères (8) d'instruments détectables par ladite gamma-caméra (3).

2. Système de suivi médical selon la revendication 1, comprenant un système de localisation électromagnétique créant un champ électromagnétique dans lequel des objets peuvent être localisés, ledit champ électromagnétique définissant ledit ou un système de coordonnées.

3. Système de suivi médical selon l'une quelconque des revendications 1 et 2, dans lequel ledit ou un système de coordonnées est défini d'après des positions déterminées de ladite gamma-caméra (3) et/ou d'un ou de plusieurs desdits instruments localisés (5, 9).

4. Système de suivi médical selon l'une quelconque des revendications 1 à 3, dans lequel ladite gamma-caméra (3) porte des repères de suivi à l'aide desquels elle peut être localisée dans l'espace et/ou identifiée dans ledit système de suivi.

5. Système médical de navigation comprenant un système de suivi selon une plusieurs des revendications précédentes.

6. Système médical de navigation selon la revendications 5, comprenant un dispositif de mémoire stockant des images obtenues par ladite gamma-caméra (3).

7. Système médical de navigation selon la revendication 5 ou 6, comprenant une sortie d'image pour afficher des images obtenues par ladite gamma-caméra (3), en particulier conjointement avec un ou plusieurs des éléments suivants :
- des données d'images de patient à position attribuée ou repérée dans l'espace, par exemple des données de tomodensitométrie ou de résonance magnétique ;
- des instruments à position attribuée ou repérée dans l'espace, ou leurs données de localisation ;
- des instruments localisés par ladite gamma-caméra ; et
- ladite gamma-caméra elle-même.
